# FASCICULE DE BREVET EUROPEEN

(11) **EP 1 110 529 B1**
(45) Date de publication et mention de la délivrance du brevet: **07.10.2009**
(21) Numéro de dépôt: 00420265.1
(22) Date de dépôt: 20.12.2000
(51) Int. Cl.: A61F 13/56

(54) **Couche-culotte munie d'une ceinture arrière à replis lateraux**
Auf den hinteren Teil eines absorbierenden Artikels befestigter Gürtel mit gefalteten Seitenenden
A belt with folded ends attached on the backside of an absorbent article

(30) Priorité: 23.12.1999 FR 9916407
(43) Date de publication de la demande: 27.06.2001
(73) Titulaire: Hygiene-Medica, 34400 Lunel Viel (FR)
(72) Inventeur: Brutin, Jean-Marc, 59780 Camphin en Pevele (FR); Lorthioit, Bruno, 59171 Hornaing (FR); Comte, Tony, 69220 Saint Jean d'Ardieres (FR)
(74) Mandataire: Feray, Valérie

(56) Documents cités:
- EP-A- 0 211 197
- WO-A-95/29657
- WO-A-98/22069
- WO-A-99/21522
- US-A- 4 024 867
- US-A- 4 537 591

## Description

La présente invention concerne une couche-culotte à jeter comprenant une feuille extérieure imperméable, une feuille intérieure perméable et un matelas absorbant interposé entre les deux dites feuilles; elle concerne plus particulièrement une couche-culotte munie d'une bande de ceinture fixée transversalement sur la partie arrière de la feuille extérieure et dont les extrémités comportent des dispositifs d'attaches, pour la fermeture de la couche-culotte.

Une couche-culotte munie d'une bande de ceinture arrière est connue notamment par les documents EP 0 206 208 A1 et EP 0 211 197 A1.

Dans ces deux documents, il s'agit d'une bande de ceinture réalisée dans un matériau élastique ou dans un matériau rétractable à chaud et capable après rétraction, de présenter des caractéristiques d'élasticité. Dans le premier document EP 0 206 208 A1, la bande de ceinture est fixée à la feuille extérieure uniquement dans la zone médiane de celle-ci ; de plus, elle est disposée dans un gousset formé entre un élément de recouvrement et la feuille extérieure, en laissant subsister un bord extrême libre de la bande de ceinture au-delà de la partie arrière, pour sa préhension par l'utilisateur. Dans le document EP 0 211 197 A1, la bande de ceinture qui est réalisée en matériau rétractable à chaud, est fixée à la feuille extérieure à l'état non rétracté pendant la fabrication de la couche-culotte, le traitement permettant d'obtenir la rétraction étant réalisé après fixation de la bande de ceinture.

Notamment, la bande de ceinture est fixée sur toute la largeur de la feuille extérieure par une pluralité de lignes longitudinales d'un matériau adhésif déposé à chaud et conservant ses qualités adhésives après refroidissement ou bien par des soudures réalisées par application d'ultra sons ou par calandrage.

Dans un mode particulier de réalisation, similaire dans les deux documents précités, il est prévu que la feuille extérieure soit de forme globalement rectangulaire de relative faible largeur de telle sorte que la bande de ceinture dépasse largement de chaque côté de la feuille extérieure. Dans ce cas, lorsque la couche-culotte est mise en place sur l'utilisateur, le passage des jambes reste ouvert, puisque la feuille extérieure est exempte d'oreilles, c'est-à-dire des prolongements latéraux dans les parties avant et arrière de la couche qui a alors la forme générale d'un sablier. Seule la bande de ceinture permet la liaison des parties avant et arrière au niveau des hanches de l'utilisateur. Cette variante de réalisation permet une économie de matière par la suppression des oreilles et également de standardiser la fabrication dans la mesure où, pour certaines gammes de taille, il suffit d'augmenter la longueur de la ceinture et/ou la longueur de la couche-culotte.

Cependant, une difficulté apparaît pour ce qui concerne la présentation de la couche-culotte ainsi munie d'une bande de ceinture de grande longueur, rien n'étant prévu à cet égard dans les documents EP 0 206 208 A1 et EP 0 211 197 A1.

On connaît par ailleurs, d'après le document US-4 024 867, une couche-culotte dont les extrémités libres de la bande de ceinture sont repliées en direction de l'intérieur de la couche-culotte. Même si ces dispositions permettent d'améliorer la présentation de la couche, il est toutefois difficile, pour un utilisateur de se saisir de ces extrémités libres repliées. De plus, la fabrication d'une telle couche est rendue plus compliquée.

Ces difficultés sont résolues par la couche-culotte de l'invention qui comprend, une feuille extérieure imperméable, une feuille intérieure perméable et un matelas absorbant interposé entre les deux dites feuilles ; cette couche-culotte comprend également, de manière connue par le document US-4 024 867, une bande de ceinture fixée transversalement sur la partie arrière de la feuille extérieure et des dispositifs d'attache fixés sur les extrémités de la bande de ceinture, la longueur totale (L) au repos de la bande de ceinture étant supérieure à la largeur (l) de ladite feuille extérieure, chaque partie extrême libre de la bande, c'est-à-dire non fixée à la feuille extérieure, y compris le dispositif d'attache attenant, étant repliée en un seul repli latéral, le maintien à plat de chaque repli de la bande de ceinture, étant obtenu grâce à la coopération des dispositifs d'attache.

L'objet de l'invention est défini dans la revendication 1.

Ainsi, grâce aux replis latéraux, maintenus à plat lors des opérations de pliage et d'empaquetage des couches-culottes, les parties extrêmes libres de la bande de ceinture ne sont pas susceptibles de former des plis rédhibitoires et de préférence ne génèrent pas d'encombrement supplémentaire par rapport aux dimensions de la feuille extérieure, et sont facilement préhensibles par un utilisateur. De plus, les moyens de solidarisation pour le maintien à plat du ou des replis le long de la portion de la bande de ceinture fixée à la feuille extérieure n'étant que temporaires, la bande de ceinture peut aisément être déployée lors de la mise en place de la couche-culotte sur l'utilisateur.

Les dispositifs d'attache et les moyens de solidarisation temporaire sont du type à crochets ou à boucles, s'agissant d'un système d'attache mécanique auto-aggripant.

Avantageusement, la ligne de pliage du premier repli est formée à proximité immédiate de la portion fixée de la bande. Cette première ligne de pliage peut être soit au ras du bord longitudinal de la feuille extérieure soit en retrait par rapport à celui-ci.

La portion extrême libre de la bande de ceinture est, de préférence, élastique ou extensible, tandis que la portion fixée de ladite bande de ceinture n'est ni élastique ni extensible. Ainsi, l'ajustement de la couche-culotte autour de la taille de l'utilisateur se fait exclusivement par les portions extrêmes libres de la bande de ceinture.

Dans le cas où ce sont les seuls dispositifs d'attache qui permettent ce maintien à plat, lesdits dispositifs d'attache font office de moyens de solidarisation temporaire. On comprend que la longueur des parties extrêmes libres de la bande de ceinture, incluant les dispositifs d'attache attenants, est alors choisie en fonction de la longueur de la portion fixée de la bande, pour obtenir l'effet de maintien à plat.

De préférence, les dispositifs d'attache et les moyens de solidarisation temporaire étant des éléments du type à boucles (ou à crochets), la longueur des parties extrêmes libres est déterminée en sorte que l'extrémité d'un repli latéral donné, portant lesdits éléments à boucles (ou à crochets) chevauche l'extrémité de l'autre repli latéral portant des éléments à crochets (ou à boucles). Il est à noter que selon cette variante de réalisation, les éléments à boucles et à crochets qui coopèrent pour obtenir le maintien à plat des deux replis latéraux contre la portion fixée de la bande de ceinture peuvent également être mis à profit pour la fermeture de la couche-culotte, enroulée sur elle-même, après utilisation pour être mise au rebut.

Pour la fermeture de la couche-culotte autour du corps de l'utilisateur, deux cas de figure sont possibles. Selon le premier cas de figure, les dispositifs d'attache fixés aux extrémités libres de la bande de ceinture coopèrent avec des dispositifs complémentaires d'attache portés sur la portion avant de la couche-culotte. Selon le second cas de figure, les dispositifs d'attache fixés aux deux extrémités libres de la bande de ceinture coopèrent ensemble pour réaliser cette fermeture.

La présente invention sera mieux comprise à la lecture de la description qui va être faite de plusieurs exemples de réalisation d'une couche-culotte à jeter munie d'une bande de ceinture arrière avec replis latéraux, illustrés par les dessins annexés dans lesquels :
- la figure 1 est une vue schématique en perspective d'une couche-culotte dont la bande de ceinture comporte, selon chaque partie extrême libre, deux replis en forme de « Z »,
- la figure 2 est une représentation schématique en coupe d'une couche-culotte dont les parties extrêmes libres de la bande de ceinture ne présentent qu'un seul repli latéral, les deux replis latéraux étant solidarisés l'un à l'autre.

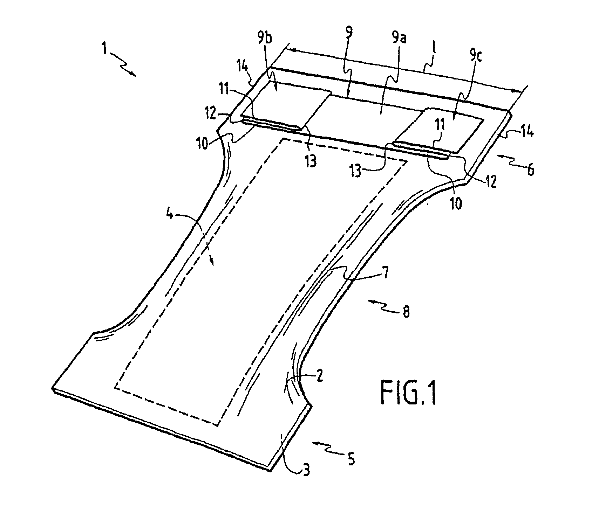

Une couche-culotte 1, à jeter, destinée aux enfants en bas âge ou aux incontinents adultes, est constituée d'une feuille extérieure 2, imperméable, d'une feuille intérieure 3 perméable et d'un matelas absorbant 4 qui est pris en sandwich entre la feuille extérieure 2 et la feuille intérieure 3. Dans l'exemple illustré à la figure 1, la feuille extérieure a une configuration générale en sablier avec des prolongements latéraux, généralement dénommés « oreilles », dans les parties avant 5 et arrière 6 de la couche 1. De plus, des élastiques 7 sont posés à l'état tendu le long du matelas absorbant 4 dans les zones 8 d'entrejambes situées entre les parties avant 5 et arrière 6. Cette structure générale de la couche-culotte 1 est montrée à titre d'exemple non exhaustif, en particulier, dans le cadre de l'invention, la couche peut ne pas présenter de prolongements latéraux du type « oreilles » dans les parties avant 5 et arrière 6.

Cette couche 1 est munie d'une bande de ceinture 9 (de longueur L) qui est fixée sur la feuille extérieure 2, transversalement à celle-ci, dans la partie arrière 6 de la couche 1 (de largeur I). Cette bande 9 présente, au-delà de la portion 9a qui est fixée sur la feuille extérieure 2, deux parties extrêmes 9b, 9c dont les extrémités libres sont équipées de dispositifs d'attache non représentés sur la figure 1. De manière caractéristique, les deux parties extrêmes 9b, 9c de la bande de ceinture 9 sont repliées à plat, en un seul ou en plusieurs replis (deux replis 10 et 11 illustrés sur la figure 1) sur la portion fixée 9a de la bande de ceinture 9, de manière à s'étendre au-dessus des extrémités fixées de cette portion fixée 9a. Plus précisément, dans ce mode de réalisation tel qu'illustré à la figure 1, les deux replis 10, 11 sont formés grâce à deux lignes de pliage 12, 13 formées dans les parties extrêmes 9b, 9c de la bande de ceinture 9. La portion 9a de la bande de ceinture a été fixée sur quasiment toute la largeur l de la couche 1 dans la partie arrière 6 de la feuille extérieure 2, de sorte que les deux premières lignes de pliage 12 se trouvent à proximité immédiate des deux bords longitudinaux 14 de la partie arrière 6 de la couche 1. Les secondes lignes de pliage 13 sont réalisées sensiblement à la moitié de la longueur des parties extrêmes libres 9b, 9c, de la bande de ceinture 9. Des moyens de solidarisation temporaire, non montrés sur la figure 1, permettent d'obtenir le maintien à plat des deux replis 10, 11 le long de la portion fixée 9a de la bande 9 sur la feuille extérieure 2. Les deux replis 10, 11 sont ainsi formés en direction de l'extérieur de la couche-culotte, de manière à s'étendre à l'opposé de la feuille extérieure 2 par rapport à la portion fixée 9a de la bande de ceinture 9. Les extrémités fixées de cette portion fixée 9a s'étendent donc entre les premiers replis 10 et la feuille extérieure 2. Les parties extrêmes 9b et 9c sont en quelque sorte repliées en forme « d'accordéon ».

Comme indiqué précédemment, il est possible d'utiliser tout type de dispositif d'attache. S'agissant en particulier de moyens d'attache mécanique par emboîtement, il suffit de fixer sur l'extrémité libre de la première partie extrême 9b de la bande 9 un organe mâle et sur l'extrémité libre de la seconde partie extrême 9c un organe femelle, l'organe mâle étant apte à pénétrer et à se bloquer en position par emboîtement dans l'organe femelle.

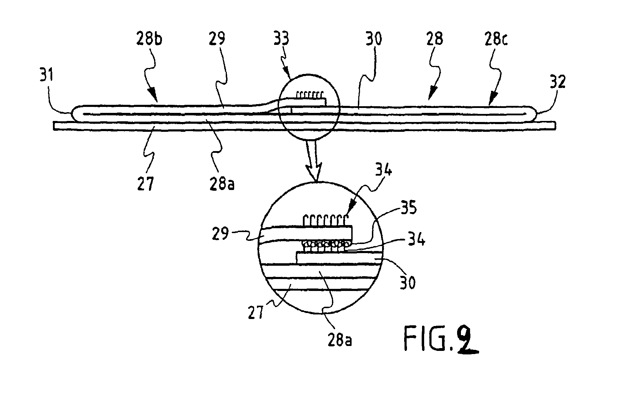

Sur la figure 2 a été représenté un exemple de réalisation dans lequel sur la feuille extérieure 27 de la couche-culotte est fixée une bande de ceinture 28 qui comprend une portion fixée 28a et deux parties extrêmes libres 28b, 28c, chacune d'elles étant repliée sous la forme d'un seul repli latéral respectivement 29 et 30. Les deux replis latéraux 29, 30 ont une longueur telle, au-delà des lignes de pliage 31, 32, qu'ils se chevauchent dans une zone centrale 33 qui est représentée en agrandissement sur cette même figure 9. Dans cette zone centrale de chevauchement 33, sont disposés à la fois les dispositifs d'attache pour la fermeture de la couche et d'autre part les moyens de solidarisation temporaire des deux replis latéraux 29, 30. Plus précisément, les dispositifs d'attache sont constitués par des éléments à crochets 34 portés sur la face externe des deux replis latéraux 29, 30, tandis que les moyens de solidarisation temporaire consistent dans un élément à boucles 35 porté par la face interne du premier repli latéral 29, ledit élément à boucles 35 coopérant avec l'élément à crochets 34 du second repli latéral 30. Ainsi, selon cette configuration particulière, il n'y a qu'un seul moyen de solidarisation temporaire pour les deux replis 29 et 30, qui en l'espèce coopèrent avec l'un des dispositifs d'attache. La longueur des replis latéraux 29, 30 est déterminée pour obtenir une configuration sensiblement à plat des deux parties extrêmes correspondantes 28b, 28c sur la portion fixée 28a de la bande 28, au-delà des deux lignes de pliage 31, 32. Dans ce cas également, il est possible d'envisager la coopération d'un dispositif d'attache et du moyen de solidarisation temporaire pour obtenir la fermeture de la couche, après repliement de celle-ci, en vue de sa mise au rebut.

Bien sur, la disposition des dispositifs d'attache et des moyens de solidarisation temporaire, qui vient d'être décrit, n'est pas limitatif de l'invention, le positionnement des éléments à boucles pouvant être inversé pour obtenir le même effet.

La largeur de la ceinture peut varier en fonction notamment des tailles de couches et des matériaux utilisés, par exemple entre 20 et 150 mm.

## Revendications

1. Couche-culotte (1) comprenant une feuille extérieure imperméable (2), une feuille intérieure perméable (3) et un matelas absorbant (4) interposé entre les deux dites feuilles (2, 3), une bande de ceinture (9, 28) fixée transversalement sur la partie arrière (6) de la feuille extérieure (2) et des dispositifs d'attache fixés sur les extrémités de la bande de ceinture (28), la longueur totale (L) au repos de la bande de ceinture (28) étant supérieure à la largeur (I) de ladite feuille extérieure (2), et chaque partie extrême libre (28b, 28c) de la bande (28), c'est-à-dire non fixée à la feuille extérieure (2), y compris le dispositif d'attache attenant, étant repliée en un seul un repli latéral (29, 30), le maintien à plat de chaque repli (29, 30) de la bande (28) de ceinture étant obtenu grâce à la coopération desdits dispositifs d'attache, **caractérisé en ce que** ledit repli latéral (29, 30) est replié au dessus de la portion (28a) de la bande fixée à la feuille extérieure (2), en étant rabattu vers l'extérieur de la couche-culotte, à l'opposé de la feuille extérieure (2) par rapport à la portion fixée (28a) de la bande (28), et est maintenu à plat le long de ladite portion fixée (28a) et **en ce que** la longueur des parties extrêmes libres (28b, 28c) est déterminée en sorte que l'extrémité d'un repli latéral donné (29), portant lesdits dispositifs d'attache et des moyens de solidarisation temporaire (34, 35) chevauchent l'extrémité de l'autre repli latéral (30) portant lesdits dispositifs d'attache (34) lesdit dispositifs d'attache et les moyens de solidarisation temporaire étant des éléments du type à boucles ou à crochets.

2. Couche-culotte selon l'une des revendications 1, **caractérisée en ce que** la ligne de pliage du repli est formée à proximité immédiate de la portion fixée de la bande.

3. Couche-culotte selon l'une des revendications 1 ou 2, **caractérisée en ce que** la ligne de pliage du premier repli est soit au ras du bord longitudinal de la feuille extérieure soit en retrait par rapport à celui-ci.

4. Couche-culotte selon l'une des revendications 1 à 3, **caractérisée en ce que** la portion extrême libre de la bande de ceinture est élastique ou extensible, tandis que la portion fixée de ladite bande de ceinture n'est ni élastique ni extensible.

5. Couche-culotte selon l'une des revendications 1 à 4, **caractérisée en ce que** les dispositifs d'attache fixés aux extrémités libres de la bande de ceinture coopèrent avec des dispositifs complémentaires d'attache portés sur la portion avant de la couche-culotte.

6. Couche-culotte selon l'une des revendications 1 à 5, **caractérisée en ce que** les dispositifs d'attache fixés aux deux extrémités libres de la bande de ceinture coopèrent ensemble pour réaliser la fermeture de la ceinture.

## Claims

1. A pull-up diaper (1) comprising an impermeable external sheet (2), a permeable internal sheet (3), and an absorbent pad (4) sandwiched between said two sheets (2, 3), a waist band (9, 28) transversely fastened to the back part (6) of the external sheet (2), and fasteners fastened to the ends of the waist band (28), the total resting length (L) of the waist band (28) being greater than the width (1) of said external sheet (2), and each free end part (28b, 28c) of the band (28), i.e. not fastened to the external sheet (2), including the adjacent attaching device, being folded in a single lateral fold (29, 30), maintaining the flatness of each fold (29, 30) of the waist band (28) being obtained through the cooperation of said fasteners,
**characterized in that** said side fold (29, 30) is folded above the portion (28a) of the band fastened to the external sheet (2), by being folded back outwards of the pull-up diaper, opposite the external sheet (2) with respect to the fixed portion (28a) of the band (28), and is maintained flat along said fixed portion (28a), and **in that** the length of the free end parts (28b, 28c) is determined so that the end of a given side fold (29), bearing said fasteners and means for a temporary connection (34, 35), overlaps the end of the other side fold (30) bearing said fasteners (34), with said fasteners and means for a temporary connection being loop and hook type elements.

2. The pull-up diaper according to claim 1,
**characterized in that** the fold line of the fold is formed in close proximity of the fixed portion of the band.

3. The pull-up diaper according to any of claims 1 or 2, **characterized in that** the fold line of the first fold is located either at the level of the longitudinal edge of the external sheet or recessed with respect thereto.

4. The pull-up diaper according to any of claims 1 to 3, **characterized in that** the free end portion of the waist band is elastic or extensible, while the fixed portion of said waist band is neither elastic nor extensible.

5. The pull-up diaper according to any of claims 1 to 4, **characterized in that** the fasteners fastened to the free ends of the waist band cooperate with complementary fasteners carried on the front portion of the pull-up diaper.

6. The pull-up diaper according to any of claims 1 to 5, **characterized in that** the fasteners fastened to both free ends of the waist band cooperate with each other in order to ensure closing of the waist.

## Patentansprüche

1. Windelhose (1) umfassend eine undurchlässige Außenfolie (2), eine durchlässige Innenfolie (3) und eine absorbierende Einlage (4), die zwischen die beiden Folien (2, 3) gelegt wird, einen Taillenbund (9, 28), der quer an dem hinteren Teil (6) der Außenfolie (2) befestigt ist, und Befestigungselemente, die an den Enden des Taillenbundes (28) befestigt sind, wobei die gesamte Länge (L) im Ruhezustand des Taillenbundes (28) größer ist als die Breite (1) der Außenfolie (2), und wobei jeder freie Endteil (28b, 28c) des Bundes (28), d.h. der nicht an der Außenfolie (2) befestigt ist, einschließlich des angrenzenden Befestigungselements, zu einer einzigen Seitenfalte (29, 30) gefaltet ist, wobei das Flachhalten jeder Falte (29, 30) des Taillenbundes (28) durch das Zusammenwirken der Befestigungselemente erreicht wird,
**dadurch gekennzeichnet, dass** die Seitenfalte (29, 30) oberhalb des Abschnitts (28a) des Bundes, der an der Außenfolie (2) befestigt ist, gefaltet wird, indem sie zum Äußern der Windelhose umgebogen wird, gegenüber der Außenfolie (2) im Verhältnis zu dem festen Abschnitt (28a) des Bundes (28), und an dem festen Abschnitt (28a) entlang flach gehalten wird, und dass die Länge der freien Endteile (28b, 28c) derart bestimmt wird, dass das Ende einer bestimmten Seitenfalte (29), das die Befestigungselemente und Mittel zum vorläufigen Festmachen (34, 35) umfasst, das Ende der anderen Seitenfalte (30) überlappt, das die Befestigungselemente (34) trägt, wobei die Befestigungselemente und die Mittel zum vorläufigen Festmachen klettartige Elemente sind.

2. Windelhose nach Anspruch 1, **dadurch gekennzeichnet, dass** die Faltlinie der Falte in unmittelbarer Nähe des festen Abschnitts des Bundes gebildet ist.

3. Windelhose nach einem der Ansprüche 1 oder 2,
**dadurch gekennzeichnet, dass** die Faltlinie der ersten Falte sich entweder auf Höhe des Längsrandes der Außenfolie oder im Verhältnis dazu zurückversetzt befindet.

4. Windelhose nach einem der Ansprüche 1 bis 3, **dadurch gekennzeichnet, dass** der freie Endabschnitt des Taillenbundes elastisch oder dehnbar ist, während der feste Abschnitt des Taillenbundes weder elastisch noch dehnbar ist.

5. Windelhose nach einem der Ansprüche 1 bis 4, **dadurch gekennzeichnet, dass** die Befestigungselemente, die an den freien Enden des Taillenbundes befestigt sind, mit ergänzenden Befestigungselementen zusammenwirken, die auf dem Vorderabschnitt der Windelhose getragen werden.

6. Windelhose nach einem der Ansprüche 1 bis 5, **dadurch gekennzeichnet, dass** die Befestigungselemente, die an den beiden freien Enden des Taillenbundes befestigt sind, zusammenwirken, um den Verschluss des Bundes herzustellen.
